# EUROPEAN PATENT SPECIFICATION

(11) **EP 2 576 447 B1**
(45) Date of publication and mention of the grant of the patent: **24.02.2016**
(21) Application number: 11723386.6
(22) Date of filing: 19.05.2011
(51) Int. Cl.: C01G 9/02, C01G 19/02, C01G 23/047, C01G 23/08, C09C 1/04, C09C 1/36, C09C 3/06, C09C 3/08, C09C 3/12, A61K 8/11, A61K 8/27, A61K 8/29, A61Q 17/04, B01J 13/22, A61K 8/81, A61K 8/02, B82Y 30/00

(54) **MICROSPHERES AND PHOTOPROTECTIVE PERSONAL CARE COMPOSITION COMPRISING SAME**
MIKROKÜGELCHEN UND LICHTSCHUTZ-KÖRPERPFLEGEZUSAMMENSETZUNG
MICROSPHÈRES ET COMPOSITION DE SOINS PERSONNELS PHOTOPROTECTEURS LES COMPRENANT

(30) Priority: 24.08.2010 EP 10173855; 04.06.2010 IN MU17152010
(43) Date of publication of application: 10.04.2013
(73) Proprietor: Unilever N.V., 3013 AL Rotterdam (NL); Unilever PLC, London, Greater London EC4Y 0DY (GB)
(72) Inventor: GHOSH DASTIDAR, Sudipta, Bangalore 560 066 (IN); PALANISAMY, Bharath, Bangalore 560 066 (IN)
(74) Representative: Corsten, Michael Allan
(86) International application number: PCT/EP2011/058098
(87) International publication number: WO 2011/151184

(56) References cited:
- WO-A1-02/074431
- WO-A2-2009/104011
- US-A1- 2009 155 371
- US-B1- 6 534 044
- KIMBERLY NELSON AND YULIN DENG: "Enhanced Light Scattering from Hollow Polycrystalline TiO2 Particles in a Cellulose Matrix", LANGMUIR, vol. 24, 8 January 2008 (2008-01-08), pages 975-982, XP002619749,
- ZHANG H ET AL: "Facile synthesis of monodisperse polymer/SiO2/polymer/TiO2 tetra-layer microspheres and the corresponding double-walled hollow SiO2/TiO2 microspheres", JOURNAL OF COLLOID AND INTERFACE SCIENCE, ACADEMIC PRESS, NEW YORK, NY, US, vol. 348, no. 2, 28 April 2010 (2010-04-28), pages 431-440, XP027098287, ISSN: 0021-9797 [retrieved on 2010-04-28]
- ZHENG R ET AL: "A general protocol to coat titania shell on carbon-based composite cores using carbon as coupling agent", JOURNAL OF SOLID STATE CHEMISTRY, ORLANDO, FL, US, vol. 182, no. 5, 1 May 2009 (2009-05-01), pages 1235-1240, XP026057844, ISSN: 0022-4596, DOI: DOI:10.1016/J.JSSC.2009.01.032 [retrieved on 2009-02-05]

## Description

### Technical Field

The invention relates to a photoprotective personal care composition comprising microspheres, and a process to prepare them.. In particular, the invention relates photoprotective personal care compositions that are effective in protecting the skin against harmful solar radiation especially from the visible rays while ensuring a highly acceptable even skin tone and appearance.

### Background of the invention

Highly pleasing skin appearance is one of the most desired expectations from cosmetic products from most consumers around the world. In tropical countries where consumers generally have dark skin, there is a desire to have lighter skin appearance. In consumers who live far from the tropical countries e.g. the Caucasian people who generally have lighter skin, there is a need among such consumers to have an even tanned tone of their skin. Any exposure of the skin to sunlight, in such consumers often leads to blochy skin, referred to as freckles and in some cases they experience hyperpigmentation in localized areas of the skin. Most consumers experience blemishes on their skin after exposure to sun, on healing of wounds or after drying up of acne. In all of the above cases, consumers rely on cosmetic solutions to their skin appearance problems.

Smooth, soft and glowing skin with even skin tone and colour is thus desired by all consumers who use cosmetic compositions for their skin. To provide this benefit, manufacturers from around the world have tried many approaches. One very commonly used approach is to include sunscreens or sunblocks in such cosmetic products. Sunscreens or sunblocks may be organic compounds or inorganic compounds. Sunscreens are generally organic compounds that work by absorbing ultra-violet (uv) radiation from the sun at specified wavelength range thus not permitting the uv radiation from reaching the skin surface. UV radiation is believed to be the cause of skin coloration or tanning and if such tanning is uneven, it is disliked by the consumer. Sunblocks are generally inorganic compounds that act as physical barrier against a wide range of radiation from the sun (both uv and visible light). There are some disadvantages in both these approaches. Organic sunscreens are generally effective only against specific wavelength ranges i.e. they are not broad spectrum and therefore more than one sunscreens are often to be used. There are also questions about the stability of these sunscreens on exposure to the sun. Inorganic sunblocks, while being broad spectrum, often are white in colour and leave a pale whitish appearance on the skin which is unnatural and not liked by consumers.

Another approach used by cosmetic researchers is to provide instant appearance benefit by incorporating tailor made materials or particles in cosmetics. Such materials or particles optically interact with the light incident on the skin and reflect light in such a wavelength range that makes the skin appear to have a desired colour, tone and evenness.

One or a combination of the above approaches is used in many cosmetic products. In order to provide all or most of the benefits, many different ingredients, each having a specific benefit and working through a specific mechanism need to be incorporated in the skin compositions. Some of these ingredients may interact with each other or may be unstable in the cosmetic base.

In order to provide a solution to the above problem the present inventors have been working for many years on developing tailor made materials that work through multiple routes to provide most of the skin cosmetic benefits in a single material. In the present invention the inventors have developed microspheres that combine the unique benefits of photoprotective materials and materials that provide instant optical benefits to deliver a product having benefits not achieved before.

Microspheres which can be used in cosmetics are disclosed in "Enhanced Light Scattering from Hollow Polycrystalline Ti02 Particles in a Cellulose Matrix", LANGMUIR, vol. 24. 8 January 2008, pages 975-982.

US 2009/155371 A1 (Sojka Milan) discloses a photoprotective personal care composition which comprises microspheres in combination with a cosmetically acceptable base. The microspheres contain a collapsed polymeric shell which has solid particles entrapped inside.

WO 02/074431 (Max Planck) relates to a preparation of monodisperse hollow titania spheres with defined diameter, wall thickness and crystal phase. The hollow spheres have been produced by the layered deposition of water-soluble titania precursor onto submicron sized template particles, e.g. polystyrene particles, followed by calcination at elevated temperatures.

US 2009/0155371 (Sojka) discloses topical compositions containing solid particles that are stabilized via entrapment by microspheres, each microshpere containing a collapsed polymeric shell that has entrapped therein one or more solid particles.

US 6 534 044 (Showa Denko KK) discloses a cosmetic material comprising silica coated metal oxide particles further surface coated with a hydrophobizing agent.

The present inventors have developed a microsphere with a hollow interior and a shell of a material having a specific optical property and specific thickness and coated with another material having a different specific optical property, a combination of which gives the microsphere surprising benefits both in terms of protection from the harmful sun rays while giving a pleasing skin appearance when these microspheres are incorporated in topical compositions. This microsphere has also been prepared by a novel process that gives the material these unique properties, through a simple and easy to scale up process.

It is thus an object of the present invention to provide for a material that when incorporated in a photoprotective personal care composition for giving the combined benefits of photoprotection over a wide range of wavelengths while at the same time giving the skin a pleasing even appearance.

It is another object of the present invention to provide for a process to prepare a material that can be incorporated in personal care compositions for both photoprotection over a wide range of wavelengths and pleasing even appearance to the skin where the cosmetic is applied.

It is yet another object of the present invention to provide for a material which can be incorporated in personal care compositions which give the combined benefits of wide spectrum photoprotection and benefits of instant lightening of the skin for dark skinned consumers without the skin appearing unnaturally white and pale.

### Summary of the invention

According to one aspect of the present invention there is provided a photoprotective personal care composition comprising
(a) a microsphere of 100 to 600 nm mean diameter comprising
   (i) a coated shell; and
   (ii) a hollow core comprising air;
      refractive index in the range of 1.3 to 1.6 ; said coating material of thickness in the range of 10 to 30 nm; and
(b) a cosmetically acceptable base.

According to another aspect of the present invention there is provided a process to prepare microspheres of the invention for inclusion in photoprotective personal care compositons comprising the steps of
(i) taking hollow polymeric microspheres in a solvent;
(ii) reacting a precursor of the metal oxide in the solvent phase to form metal oxide which forms a layer on the hollow polymeric microspheres thereby forming layered microspheres;
(iii) separating the layered microspheres from the solvent;
(iv) calcining the layered microspheres to prepare hollow metal oxide microspheres.
(v) preparing a dispersion of the hollow metal oxide microspheres in a solvent along with the coating material having a refractive index in the range of 1.3 to 1.6;
(vi) stirring for a period of 0.5 to 4 hours;
(vii) separating coated microspheres from the solvent.

### Brief description of the figures

The invention is described in more detail hereinbelow with reference to the figures in which:
Figure 1(a) shows a SEM image of Sunsphere™ hollow polymeric microspheres used for preparing the hollow microspheres of the invention;
Figure 1(b) shows a SEM image of hollow microspheres of the invention prepared as per example 1;
Figure 2(a) shows transmittance spectra in the visible region of a composition as per invention (example 2) as compared to a conventional composition (example 3);
Figure 2(b) shows transmittance spectra in the UV region of a composition as per invention (example 2) as compared to a conventional composition (example 3); and
Figure 3 shows absorbance spectra of a model dye solution which demonstrate the superiority of coated microspheres for incorporation in the composition of the invention as compared to uncoated microspheres.

### Detailed description of the invention

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature, multiple preferred ranges are described in the format "from x to y", it is understood that all ranges combining the different endpoints are also contemplated.

By "photoprotective personal care composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of photoprotective sunscreen compositions include leave-on skin lotions and creams, shampoos, conditioners, shower gels, toilet bars, antiperspirants, deodorants, lipsticks, foundations, mascara, sunless tanners and sunscreen lotions. "Skin" as used herein is meant to include skin on the face and body (e.g. neck, chest, back, arms, underarms, hands, legs, buttocks and scalp), especially to the sun exposed parts thereof. The composition of the invention is also of relevance to applications on any other keratinous substrate of the human body other than skin, e.g. hair, where products may be formulated with specific aim of providing photoprotection. type. The core is hollow, i.e it merely comprises air. By the term hollow is meant that the core of the microsphere is substantially free of any solid or liquid material. Preferably the core comprises more than 90 volume percent air, more preferably more than 95 volume percent air. The shell has a mean diameter of 100 to 600 nm, more preferably 300 to 400 nm, further more preferably 300 to 350 nm. By mean diameter is meant the number average mean diameter of the particles. In this specification, the particle size distribution of commercial particles were determined using Malvern particle size analyzer. The diameter of the hollow microspheres of the invention were determined using dynamic light scattering instrument (purchased from Brookhaven) which was coupled with a Lexel 95 laser (wavelength of 488 nm). The selective mean particle diameter in these preferred ranges provides for the optimum visible light scattering to ensure the desired photoprotection while maintaining the desired skin appearance. The shell has a thickness of 20 to 100 nm, more preferably 20 to 60 nm, further more preferably 20 to 30 nm. In these selective ranges of shell thickness the advantages are to ensure the desired UV ray scattering to meet the objectives of the invention viz. optimum photoprotection and skin appearance. The shell thickness of the hollow microspheres was determined from their SEM images. Image analysis using Image Pro Plus software was used to evaluate the shell thickness from the SEM images. The shell is made of a metal oxide having a refractive index of 1.8 to 3.0, more preferably 1.9 to 2.7. Metal oxides having a refractive index in the range of 1.8 to 3.0 are necessary in order to provide enhanced efficacy of light scattering. The metal oxide from which the shell is made is preferably titanium dioxide, zinc oxide, tin oxide or cerium oxide more preferably titanium dioxide or zinc oxide. The refractive indices for the various materials mentioned in this specification are those reported in well known databases like 'The Handbook of Chemistry and Physics', Publisher, CRC Press Boca Raton, Florida.

The shell is coated with a material having a refractive index in the range of 1.3 to 1.6, more preferably 1.4 to 1.6. Coating with a material having this selective refractive index is especially useful to reduce photocatalytic activity of these microsphere particles. High photocatalytic activity of these particles is undesirable for skin applications. Further, this selective property enhances dispersion and ensures spreading of the cosmetic composition on the skin applied on. The coating with a material having a refractive index in the range of 1.3 to 1.6 is of a thickness in the range of 10 to 30 nm. It is preferred that the material coated on the shell is transparent to light in the wavelength range of 200 to 400 nm. By the term "transparent to light in the wavelength range of 200 preferred that the material coated on the shell is transparent to light in the wavelength range of 200 to 400 nm. By the term "transparent to light in the wavelength range of 200 to 400 nm" is meant that the % transmittance as a function of wavelength from 290 to 400 nm is more than 50%.

Example of materials which are useful for coating the shell are silica, aluminium hydroxide, fatty acid, silicone, polysaccharides and their derivatives. Suitable polysaccharides include, starch, cellulose, cellulose acetate and cationically modified starch. Preferred coating materials are fatty acids, silicones or celluloses. Of the coating materials those which are organic compounds are more preferred. Another useful property for selecting suitable coating materials is that they have surface energy between 20 x 10⁻³ and 50 x 10⁻³ J/m², more preferably between 30 x 10⁻³ and 40 x 10⁻³ J/m². Selecting coating materials having the above properties provide for enhanced compatibility of the microspheres in cosmetic compositions while ensuring even spreading of the compositions on the topical surface where it is applied.

Surface energy is the energy required to increase the surface area of a substance by unit area. Surface energy values mentioned in this specification are the values for materials as is mentioned in standard databases found in 'The Handbook of Chemistry and Physics' Publisher, CRC Press Boca Raton, Florida, Edited by: Brandrup, J.; Immergut, Edmund H.; Grulke, Eric A.; Abe, Akihiro; Bloch, Daniel R., 2005, John Wiley & Sons.

Without wishing to be bound by theory it is believed that the combination of the hollow core, i.e. a core comprising air, and a shell having a refractive index between 2.0 and 3.0 which is coated with a coating material having a refractive index between 1.3 and 1.6 provides for less photocatalytic activity, more transparency and better dispersion.

The composition of the invention comprises the microspheres having the property as disclosed hereinabove together with a cosmetically acceptable base. The cosmetically acceptable base is such that the composition is preferably a cream, lotion, gel or emulsion. The microspheres are preferably present in 0.1 to 10%, more preferably 1 to 5% by weight of the composition.

Cosmetic compositions may be prepared using different cosmetically acceptable emulsifying or non-emulsifying systems and vehicles. A highly suitable base is a cream. Vanishing creams are especially preferred. Vanishing cream bases generally comprise 5 to 25% fatty acid and 0.1 to 10% soap. Vanishing cream base gives a highly appreciated matty feel to the skin. C₁₂ to C₂₀ fatty acids are especially preferred in vanishing cream bases, futher more preferred being C₁₄ to C₁₈ fatty acids. The most preferred fatty acid is stearic acid. The fatty acid in the composition is more preferably present in an amount in the range of 5 to 20% by weight of the composition. Soaps in the vanishing cream base include alkali metal salt of fatty acids, like sodium or potassium salts, most preferred being potassium stearate. The soap in the vanishing cream base is generally present in an amount in the range of 0.1 to 10%, more preferably 0.1 to 3% by weight of the composition. Generally the vanishing cream base in cosmetic compositions is prepared by taking a desired amount of total fatty matter and mixing with potassium hydroxide in desired amounts. The soap is usually formed in-situ during the mixing.

The composition of the invention may additionally comprise a skin lightening agent. This skin lightening agent is preferably chosen from a vitamin B3 compound or its derivative, e.g. niacin, nicotinic acid, niacinamide, or other well known skin lightening agents, e.g. aloe extract, ammonium lactate, arbutin, azelaic acid, kojic acid, butyl hydroxy anisole, butyl hydroxy toluene, citrate esters, 3-diphenylpropane derivatives, 2,5-dihydroxybenzoic acid and its derivatives, ellagic acid, fennel extract, gluco pyranosyl-1-ascorbate, gluconic acid, glycolic acid, green tea extract, hydroquinone, 4-hydroxyanisole and its derivatives, 4-hydroxybenzoic acid derivatives, hydroxycaprylic acid, lemon extract, linoleic acid, magnesium ascorbyl phosphate, mulberry root extract, 2,4-resorcinol derivatives, 3,5-resorcinol derivatives, salicylic acid, vitamins like vitamin B6, vitamin B12, vitamin C, vitamin A, a dicarboxylic acid, resorcinol derivatives, hydroxycarboxylic acid like lactic acid and their salts, e.g. sodium lactate, and mixtures thereof. Vitamin B3 compound or its derivatives, e.g. niacin, nicotinic acid, niacinamide, are the more preferred skin lightening agent as per the invention, most preferred being niacinamide. Niacinamide, when used, is preferably present in an amount in the range of 0.1 to 10%, more preferably 0.2 to 5% by weight of the composition.

The photoprotective personal care composition may preferably additionally comprise one or more uv sunscreens. The uv sunscreens may be inorganic or organic.

A wide variety of organic sunscreen agents are suitable for use in combination with the essential ingredients of this invention. Suitable UV-A or UV-B sunscreen agents include 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl-p-aminobenzoate, 3,3,5-trimethylcyclohexylsalicylate, methylanthranilate, p-dimethyl-aminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonic benzoxazoic acid, 2-ethylhexyl-p-methoxycinnamate, butylmethoxydibenzoylmethane, 2-hydroxy-4-methoxybenzophenone, octyldimethyl-p-aminobenzoic acid and mixtures thereof. Most suitable organic sunscreen are 2-ethylhexyl-p-methoxycinnamate and butylmethoxydibenzoylmethane.

A safe and effective amount of sunscreen may be used in the compositions useful in the subject invention. The composition preferably comprises from about 0.1 % to about 10%, more preferably from about 0.1 % to about 5% of a sunscreen agent.

Useful inorganic sunblocks are also preferably used in the present invention. These include, for example, zinc oxide iron oxide, silica, such as fumed silica, and titanium dioxide.

Ultrafine titanium dioxide in either of its two forms, namely water-dispersible titanium dioxide and oil-dispersible titanium dioxide is especially suitable for the invention. Water-dispersible titanium dioxide is ultra-fine titanium dioxide, the particles of which are non-coated or which are coated with a material to impart a hydrophilic surface property to the particles. Examples of such materials include aluminium oxide and aluminium silicate.

Oil-dispersible titanium dioxide is ultrafine titanium dioxide, the particles of which exhibits a hydrophobic surface property, and which, for this purpose, can be coated with metal soaps such as aluminium stearate, aluminium laurate or zinc stearate, or with organosilicone compounds.

By "ultrafine titanium dioxide" is meant particles of titanium dioxide having an average particle size of less than 100 nm, preferably 70 nm or less, more preferably from 10 to 40 nm and most preferably from 15 to 25 nm.

By topical application to the skin of a mixture of both water-dispersible ultrafine titanium dioxide and oil-dispersible ultrafine titanium dioxide, synergistically enhanced protection of the skin against the harmful effects of both UV-A and UV-B rays is achievable.

Ultrafine titanium dioxide is the preferred inorganic sunblock agent as per this invention. The total amount of sunblock that is preferably incorporated in the composition according to the invention is from 0.1 to 5% by weight of the composition.

The composition according to the invention may also comprise other diluents. The diluents act as a dispersant or carrier for other materials present in the composition, so as to facilitate their distribution when the composition is applied to the skin. The composition of the invention preferably comprises water. Water is preferably present in 35 to 90%, more preferably 50 to 85% by weight of the composition.

Diluents other than water can include liquid or solid emollients, solvents, humectants, thickeners and powders. Examples of each of these types of vehicle, which can be used singly or as mixtures of one or more vehicles, are as follows:
Emollients such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;

Solvents such as ethyl alcohol, isopropanol, acetone, ethylene glycol monoethyl ether, diethylene glycol monobutyl ether, diethylene glycol monoethyl ether; and

Powders such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated The cosmetically acceptable base is usually from 10 to 99.9%, preferably from 50 to 99% by weight of the composition, and can, in the absence of other cosmetic adjuncts, form the balance of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents and skin healing agents.

The composition is formulated in any known format, more preferred formats being creams or lotions.

The composition of the invention may comprise a conventional deodourant base as the cosmetically acceptable carrier. By a deodorant is meant a product in the stick, roll-on, or propellant medium which is used for personal deodorant benefit, e.g. application in the underarm or any other area which may or may not contain anti-perspirant actives.

Deodorant compositions can generally be in the form of firm solids, soft solids, gels, creams, and liquids and are dispensed using applicators appropriate to the physical characteristics of the composition.

According to another aspect of the present invention there is provided a process to prepare microspheres of the invention comprising the steps of taking hollow polymeric microspheres in a solvent; (a) reacting a precursor of the metal oxide in the solvent phase to form metal oxide which forms a layer on the hollow polymeric microspheres thereby forming layered microspheres; (b) separating the layered microspheres from the solvent; (c) calcining the layered microspheres to prepare hollow metal oxide microspheres (d) preparing a dispersion of the hollow metal oxide microspheres in a solvent along with a the coating material having a refractive index in the range of 1.3 to 1.6; (e) stirring for a period of 0.5 to 4 hours; and (f) separating coated microspheres from the solvent. dispersion of the hollow metal oxide microspheres in a suitable solvent along with a desired coating material; (e) stirring for a period of 0.5 to 4 hours; and (f) separating coated microspheres from the solvent.

It is important that the polymeric microspheres for preparing the microspheres of the invention is hollow. This ensures that the microspheres of desired size and shape with the hollow core can be prepared without too many of the microspheres breaking up during the calcining process. The hollow polymeric microspheres are preferably made of polystyrene, polyacrylate or polystyrene co-polyacrylate. The hollow polymeric microspheres preferably have an inner diameter in the range of 100 to 350 nm and an outer diameter in the range of 150 to 400. The hollow polymeric microspheres are first taken in a solvent. Suitable solvents include ethanol, methanol, propanol or isopropanol. The hollow polymeric microspheres are taken in the solvent at a temperature preferably in the range of 60 to 90°C. The hollow polymeric microspheres dispersed in the solvent is then treated with a precursor of the metal oxide. Precursors of metal oxides are generally organometallic in nature, e.g. when titanium dioxide or zinc oxide are to be prepared, suitable precursors are alkoxides, sulphates, nitrates, acetates or chlorides more preferably alkoxides. The hollow polymeric microspheres coated with the desired metal oxide are then separated from the dispersion and dried. They are then calcined, preferably at a temperature in the range of 400 to 800 °C to form hollow metal oxide microspheres.

The hollow metal oxide microspheres are then coated with a coating material having a refractive index in the range of 1.3 to 1.6. Suitable coating materials are cellulose, cellulose acetate, starch or cellulose ethers e.g. hydroxypropyl cellulose. The coating process preferably comprises the steps of (i) preparing a dispersion of the hollow metal oxide microspheres in a suitable solvent along with the desired coating material; (ii) stirring for a period of 0.5 to 4 hours; and (iii) and separating the coated microspheres from the solvent. The separation process is preferably filtration followed by evaporation of the solvent.

Preferred solvents during the coating process are acetone or isopropyl alcohol. To this, the metal oxide particles are dispersed and stirred until the solvent is completely evaporated.

The invention is now further described by way of the following non-limiting examples.

### EXAMPLES

### Example 1 Preparation of hollow microspheres

Hollow polymeric microspheres (250 mg) made of polystyrene co-polyacrylate having outer diameter of 350 nm available as Sunsphere™ from Rohm and Haas were taken in a beaker containing 100 ml of ethanol. The hollow polymeric microspheres were suspended in ethanol through sonication for 30 minutes. To this, 0.72 ml of water was added and heated to 70 °C on an oil bath and kept stirred using a magnetic stirrer. Titanium dioxide was coated on the hollow polymeric microspheres by dropping a precursor viz. titanium butoxide (2.72 ml) on to the suspension in a dropwise manner. The reaction was continued for two hours with constant stirring. The particles so prepared were separated from the suspension by centrifugation and washed with ethanol and dried. The dried samples were calcined at 700 °C for five hours in a muffle furnace.

The hollow microspheres prepared above were coated using the following process. 25 mg of cellulose acetate (Fluka) was dissolved in 100 ml of acetone. To this, 200 mg of the hollow titania particle prepared above were suspended by sonicating the sample for 30 minutes. After sonication, the sample was stirred in a glass beaker using a magnetic stirrer until the acetone evaporated completely. The cellulose acetate coated hollow particle were dried at 50 °C.

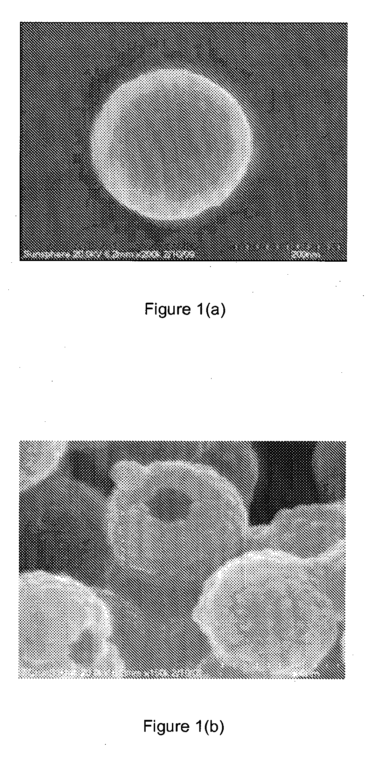

A sample of the coated hollow titania microspheres so prepared was characterized using SEM imaging in comparison to the precursor material i.e. the hollow polymeric microsphere. The SEM image of the Sunsphere™ sample is shown in figure 1a and the coated hollow metal oxide microspheres of the invention are shown in figure 1 b.

### Examples 2 and 3: Photoprotective personal care compositions of the invention (example 2) prepared with the hollow microspheres of the invention in comparision to a conventional composition (example 3)

The sample as prepared in example 1 was formulated in a photoprotective skin care composition (example 2) and compared to a control composition where the particles of example 1 was replaced with commercially available micronised titanium dioxide (example 3). The compositions of the samples of examples 2 and 3 are shown in table 1.

**Table 1**

| Ingredient | Example 2 (wt%) | Example 3 (wt%) |
|---|---|---|
| Hysteric acid | 17.00 | 17.00 |
| Hollow particles of example 1 | 2.00 | - |
| Micronised titanium dioxide | - | 2.00 |
| Glycerine | 1.00 | 1.00 |
| Isopropyl myristate | 0.75 | 0.75 |
| Potassium hydroxide | 0.57 | 0.57 |
| Cetyl alcohol | 0.53 | 0.53 |
| Silicone oil | 0.50 | 0.50 |
| Methyl paraben + propyl paraben | 0.30 | 0.30 |
| Phenoxy ethanol | 0.20 | 0.20 |
| Disodium EDTA | 0.04 | 0.04 |
| Water | To 100 | To 100 |

The micronised titanium dioxide used in example 3 was a commercial sample MT 100Z (Presperse). It is a titanium dioxide particle that is coated with aluminium hydroxide and aluminium stearate.

The samples of example 2 and 3 were compared by measuring the transmittance spectra in the UV-Vis region using the following procedure.

A transpore tape (ex. 3M) was used as a substrate to assess the efficacy of the particles. The transpore tape was stretched on a sample holder and concentration of 2 mg/cm² of the sample was dispensed uniformly using a syringe. Parafilm (ex. Pechiney Plastic Packaging, USA) was used as a finger coat and the sample was spread uniformly on the transpore tape. The film was allowed to dry for fifteen minutes before performing the measurement. The sample was exposed to a UV lamp and the transmittance spectrum in the UV region was collected using SPF-290S spectrophotometer (ex Optometrics Corporation, USA). The instrument scans six spots for a given sample. The experiment was repeated three times and the data reported is thus an average of 18 data points. The reference transmittance scan was obtained using a blank plate, with no sample on transpore tape.

For visible region, transpore tape was pasted on to a quartz plate and the same concentration (2mg/cm²) of sample was applied and spread uniformly. The sample was allowed to dry for fifteen minutes followed by exposure to Sunlamp (Atlas Suntest M/C CPS+) and the transmittance spectrum was collected using a radiometer detector (ex. International Light Technologies).

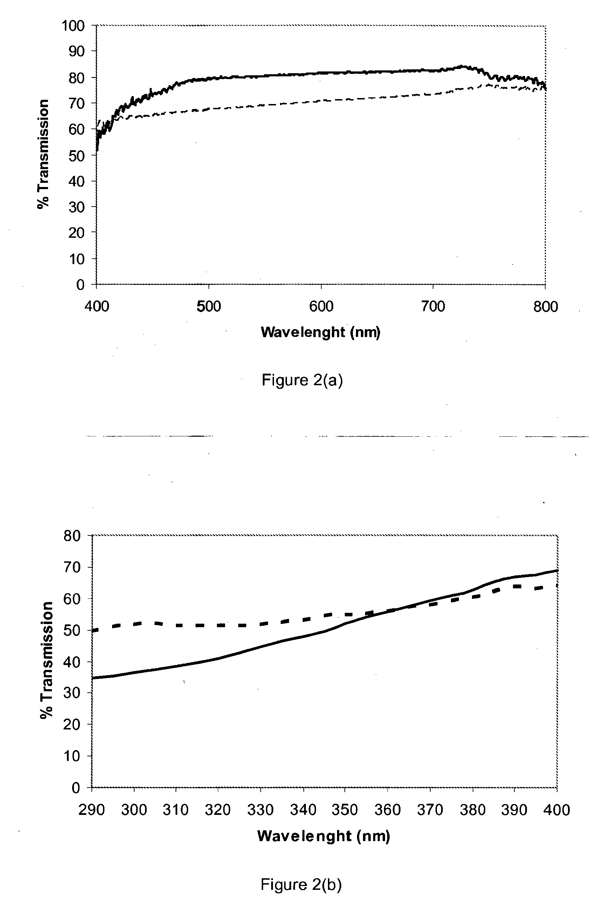

The transmission spectra of example 2 and example 3 in the visible region are shown in figure 2(a) and in the UV region in figure 2(b). The data in figures 2(a) and 2(b) indicates that hollow titania microspheres prepared as per the invention transmits less light as compared to a control composition in the visible region and is comparable in the UV region. Further, the compositions of examples 2 and 3 when applied on the skin provided almost the same skin appearance. Thus, the hollow particle of the invention provides for better visible light protection as compared to a known composition with no compromise on the UV protection and skin appearance.

### Example 4 and 5: Experiments to demonstrate the usefulness of using coated micropheres in the invention

Samples of hollow titania microspheres as per process of example 1 were prepared with coating (example 4) and without coating (example 5). It is desirable that the microspheres for use in the personal care composition of the invention have high photoprotection efficacy but low photocatalytic efficacy. The photocatalytic efficacy was measured using methylene blue (a dye used as a surrogate for organic sunscreens used in personal care compositions). Samples of particles of example 4 and 5 were suspended in water by sonication for 30 minutes. After sonication, methylene blue was added and then the samples exposed to sunlight using a solar simulator (Suntest CPS +) at 500 mW for 20 minutes. After exposure, the samples were centrifuged to remove the particles and the supernatant used for absorbance measurement. Absorbance of the supernatant was measured using a Nanodrop (ThermoScientific) spectrophotometer.

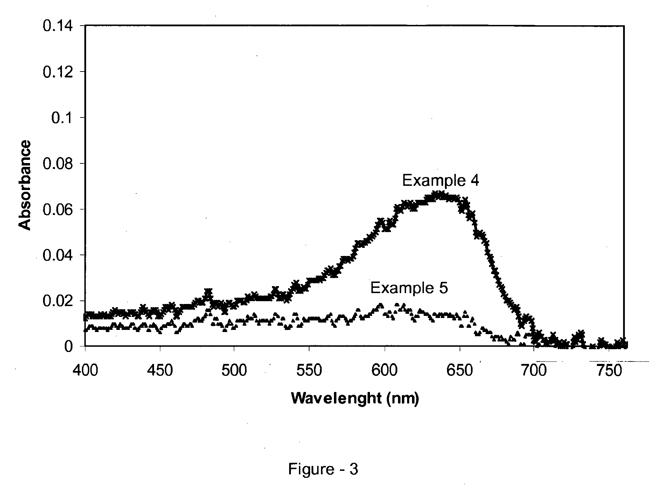

The data is presented in figure 3. The data indicates that use of microspheres as per the invention provides for enhanced stability of photosensitive compounds (e.g. organic sunscreens) used in personal care compositions.

## Claims

1. A photoprotective personal care composition comprising
(a) a microsphere of 100 to 600 nm mean diameter comprising
a. a coated shell; and
b. a hollow core comprising air;
said shell of 20 to 100 nm thickness comprising a metal oxide having a refractive index in the range of 1.8 to 3.0; said shell coated with a coating material having a refractive index in the range of 1.3 to 1.6; said coating material of thickness in the range of 10 to 30 nm; and
(b) a cosmetically acceptable base.

2. A composition as claimed in claim 1 wherein said metal oxide is titanium dioxide or zinc oxide.

3. A'compostion as claimed in claim 1 or claim 2 wherein said coating material is transparent to light in the wavelength range of 200 to 400 nm.

4. A composition as claimed in any one of the preceding claims wherein said coating material is aluminium hydroxide, fatty acid, silicone, polysaccharides or their derivatives.

5. A composition as claimed in any one of the preceding claims wherein said coating material is an organic compound.

6. A composition as claimed in any one of the preceding claims wherein said coating material has a surface energy between 20 x 10⁻³ - 50 x 10⁻³ J/m².

7. A composition as claimed in any one of the preceding claims wherein the cosmetically acceptable base is a cream, lotion, gel or emulsion.

8. A process to prepare microspheres for use in a photoprotective personal care composition according to any one of the preceding claims comprising the steps of
a. taking hollow polymeric microspheres in a solvent;
b. reacting a precursor of the metal oxide in the solvent phase to form metal oxide which forms a layer on the hollow polymeric microspheres thereby forming layered microspheres;
c. separating the layered microspheres from the solvent;
d. calcining the layered microspheres to prepare hollow metal oxide microspheres.
e. preparing a dispersion of the hollow metal oxide microspheres in a solvent along with the coating material having a refractive index in the range of 1.3 to 1.6;
f. stirring for a period of 0.5 to 4 hours;
g. separating coated microspheres from the solvent.

## Patentansprüche

1. Lichtschutzkörperpflegezusammensetzung, umfassend
(a) ein Mikrokügelchen eines mittleren Durchmessers von 100 bis 600 nm, umfassend
a. eine beschichtete Hülle und
b. einen hohlen Kern, umfassend Luft,
wobei die Hülle einer Stärke von 20 bis 100 nm ein Metalloxid mit einem Brechungsindex in dem Bereich von 1,8 bis 3,0 umfasst, die Hülle mit einem Beschichtungsmaterial eines Brechungsindexes in dem Bereich von 1,3 bis 1,6 beschichtet ist, das Beschichtungsmaterial eine Dicke in dem Bereich von 10 bis 30 nm aufweist und
(b) eine kosmetisch akzeptable Grundlage.

2. Zusammensetzung nach Anspruch 1, worin das Metalloxid Titandioxid oder Zinkoxid ist.

3. Zusammensetzung nach Anspruch 1 oder 2, wobei das Beschichtungsmaterial in dem Wellenlängenbereich von 200 bis 400 nm für Licht transparent ist.

4. Zusammensetzung nach irgendeinem der vorhergehenden Ansprüche, wobei das Beschichtungsmaterial Aluminiumhydroxid, Fettsäure, Silikon, Polysaccharide oder deren Derivate ist.

5. Zusammensetzung nach irgendeinem der vorhergehenden Ansprüche, wobei das Beschichtungsmaterial eine organische Verbindung ist.

6. Zusammensetzung nach irgendeinem der vorhergehenden Ansprüche, wobei das Beschichtungsmaterial eine Oberflächenenergie zwischen 20 x 10⁻³ - 50 x 10⁻³ J/m² hat.

7. Zusammensetzung nach irgendeinem der vorhergehenden Ansprüche, worin die kosmetisch akzeptable Grundlage eine Creme, eine Lotion, ein Gel oder eine Emulsion ist.

8. Verfahren zur Herstellung von Mikrokügelchen zur Verwendung in einer Lichtschutzkörperpflegezusammensetzung nach irgendeinem der vorhergehenden Ansprüche, umfassend die Schritte
a. Aufnehmen hohler polymerer Mikrokügelchen in ein Lösungsmittel,
b. Umsetzen einer Vorstufe des Metalloxids in der Lösungsmittelphase, um Metalloxid zu bilden, welches auf den hohlen polymeren Mikrokügelchen eine Schicht bildet, wodurch bedeckte Mikrokügelchen gebildet werden,
c. Separieren der bedeckten Mikrokügelchen aus dem Lösungsmittel,
d. Kalzinieren der bedeckten Mikrokügelchen, um hohle Metalloxid-Mikrokügelchen herzustellen,
e. Herstellen einer Dispersion der hohlen Metalloxid-Mikrokügelchen in einem Lösungsmittel zusammen mit dem Beschichtungsmaterial mit einem Brechungsindex in dem Bereich von 1,3 bis 1,6,
f. Rühren für eine Zeitdauer von 0,5 bis 4 Stunden,
g. Abtrennen der beschichteten Mikrokügelchen von dem Lösungsmittel.

## Revendications

1. Composition pour l'hygiène personnelle photoprotectrice comprenant
(a) une microsphère de 100 à 600 nm de diamètre moyen comprenant
a. une enveloppe revêtue ; et
b. un noyau creux comprenant de l'air ;
ladite enveloppe d'épaisseur de 20 à 100 nm comprenant un oxyde de métal présentant un indice de réfraction dans l'intervalle de 1,8 à 3,0 ; ladite enveloppe revêtue d'un matériau de revêtement présentant un indice de réfraction dans l'intervalle de 1,3 à 1,6 ; ledit matériau de revêtement d'épaisseur dans l'intervalle de 10 à 30 nm ; et
(b) une base cosmétiquement acceptable.

2. Composition selon la revendication 1, dans laquelle ledit oxyde de métal est le dioxyde de titane ou l'oxyde de zinc.

3. Composition selon la revendication 1 ou la revendication 2, dans laquelle ledit matériau de revêtement est transparent à de la lumière dans l'intervalle de longueur d'onde de 200 à 400 nm.

4. Composition selon l'une quelconque des revendications précédentes, dans laquelle ledit matériau de revêtement est l'hydroxyde d'aluminium, un acide gras, un silicone, des polysaccharides ou leurs dérivés.

5. Composition selon l'une quelconque des revendications précédentes, dans laquelle ledit matériau de revêtement est un composé organique.

6. Composition selon l'une quelconque des revendications précédentes, dans laquelle ledit matériau de revêtement présente une énergie de surface de 20 x 10⁻³ - 50 x 10⁻³ J/m².

7. Composition selon l'une quelconque des revendications précédentes, dans laquelle la base cosmétiquement acceptable est une crème, une lotion, un gel ou une émulsion.

8. Procédé de préparation de microsphères pour une utilisation dans une composition pour l'hygiène personnelle photoprotectrice selon l'une quelconque des revendications précédentes comprenant les étapes consistant
a. à prélever des microsphères polymères creuses dans un solvant ;
b. à faire réagir un précurseur de l'oxyde de métal dans la phase de solvant pour former un oxyde de métal qui forme une couche sur les microsphères polymères creuses formant par-là des microsphères stratifiées ;
c. à séparer les microsphères stratifiées du solvant ;
d. à calciner les microsphères stratifiées pour préparer des microsphères creuses d'oxyde de métal.
e. à préparer une dispersion des microsphères creuses d'oxyde de métal dans un solvant avec le matériau de revêtement ayant un indice de réfraction dans l'intervalle de 1,3 à 1,6 ;
f. à agiter sur une durée de 0,5 à 4 heures ;
g. à séparer les microsphères revêtues du solvant.
